# EUROPÄISCHE PATENTSCHRIFT

(11) **EP 1 140 160 B1**
(45) Veröffentlichungstag und Bekanntmachung des Hinweises auf die Patenterteilung: **22.09.2004**
(21) Anmeldenummer: 99967946.7
(22) Anmeldetag: 16.12.1999
(51) Int. Cl.: A61K 39/118, A61P 31/04

(54) **VERFAHREN ZUR HERSTELLUNG VON AUTOVAKZINEN ZUR BEHANDLUNG VON CHLAMYDIOSEN VON SÄUGETIEREN UND MENSCHEN**
METHOD FOR PRODUCING AUTOGENOUS VACCINES FOR TREATING CHLAMYDIAL INFECTIONS IN MAMMALS AND HUMANS
PRODUCTION D'AUTOVACCINS PERMETTANT DE TRAITER LES CHLAMYDIOSES CHEZ LES MAMMIFERES ET CHEZ L'HOMME

(30) Priorität: 28.12.1998 DE 19860438
(43) Veröffentlichungstag der Anmeldung: 10.10.2001
(73) Patentinhaber: Sonntag, Hans-Günter, 69245 Bammental (DE); Nolte, Oliver, 69214 Eppelheim (DE); Weiss, Hannelore, 69151 Neckargemünd (DE); Weiss, Hans-Erich, 69151 Neckargemünd (DE)
(72) Erfinder: Sonntag, Hans-Günter, 69245 Bammental (DE); Nolte, Oliver, 69214 Eppelheim (DE); Weiss, Hannelore, 69151 Neckargemünd (DE); Weiss, Hans-Erich, 69151 Neckargemünd (DE)
(74) Vertreter: Wagner, Jutta, Dr.
(86) Internationale Anmeldenummer: PCT/EP1999/009993
(87) Internationale Veröffentlichungsnummer: WO 2000/038712

(56) Entgegenhaltungen:
- DE-A- 19 916 085
- US-A- 5 242 686
- WEISS H -E ET AL: "Successful treatment of ear, nose and throat infections with Pseudomonas aeruginosa autovaccines in small animal practice: An alternative for humans as well?" TIERAERZTLICHE UMSCHAU, Bd. 53, Nr. 1, 1. Januar 1998 (1998-01-01), Seiten 38-40, 43, XP000929814 ISSN: 0049-3864

## Beschreibung

Die vorliegende Erfindung betrifft ein neuartiges Verfahren zur Herstellung von Autovakzinen zur Behandlung von Chlamydiosen von Säugetieren und Menschen.

Chlamydien sind intracellulär parasitierende pathogene Mikroorganismen. Sie vermehren sich nur in lebenden Zeilen, so z. B. im Gewebe befallener Organsysteme wie Uterus und Lungen. In abgestoßenen, zerstörten Zellschichten wie uterine Mucosa, Placenten oder Pleuraexudate sind sie massenhaft nachzuweisen (Immunfluoreszenz).

Es ist bekannt, daß Chlamydien nur ungenügend durch die körpereigenen Abwehrmechanismen im menschlichen oder tierischen Körper bekämpft werden und nur schlecht einer Chemotherapie zugänglich sind.

Chlamydien sind eine häufige Ursache für Genitalinfektionen (NGU = non gonorrhoeal urethritis) und führen bei Schafen und Rindern zu Fehlgeburten und bei Menschen teilweise zur Sterilität (Chlamydia trachomatis). Bei Hauskatzen kommt der zur Gruppe der Chlamydien gerechnete Erreger der Katzenkratzkrankheit vor, wodurch Keratoconjunctivitiden ausgelöst werden. Weiterhin wird ein ursächlicher Zusammenhang zwischen einer Chlamydia pneumonia-Infektion und Fällen coronarer Herzerkrankungen gesehen.

Aus US-P 5,242,686 ist eine Katzenvakzine bekannt, bei welcher aus einer Hinterlegungsstelle stammende Chlamydienzellen in Hühnereiern und Nierenzellkulturen vorgezüchtet und auf Eagles MEM-Medium weitergezüchtet werden, bevor die Chlamydien durch Zusatz von Chemikalien wie Ethylenimin, Formalin, Thimerosal, Glutaraldehyd, Phenol etc. oder durch Erhitzen auf 56 °C für 5 Min. oder durch UV- oder Gammastrahlung inaktiviert werden und die Mischung in eine als Vakzine geeignete Form gebracht wird. Diese Vakzine ist zur Abwehr von mutierten Chlamydieninfektionen nicht geeignet.

Aus US-P 4,328,208 sind Vakzinen bekannt, bei denen auf Hühnereiern gezüchtete Chlamydien in Phosphat-Pufferlösung suspendiert und mit Glutaraldehyd, Merthiolat und/oder Formaldehyd bei 37 °C 10 Stunden inaktiviert werden. Der Suspension wird ein Amin und ein Polyacrylamidgel zugefügt und in einer Kolloidmühle auf Injektionsfeinheit vermahlen. Die Mischung soll als intramuskuläre Injektion zur Chlamydienprophylaxe bei beliebigen Tieren eingesetzt werden.

DD 216385 beschreibt eine ähnliche Vakzine, bei der die mit Ethylenimin 20 Stunden inaktivierte Hühnerei/Chlamydiensuspension an Aluminiumhydroxydgel gebunden wird.

WO 93-04163 beschreibt die Verwendung eines aus Hamstern isolierten Chlamydia Sp-Stammes zur Herstellung von Impfstoffen gegen andere Chlamydienstämme, wobei entweder entsprechende Antikörper oder die für den Patienten nicht infektösen Hamsterchlamydien selbst oder mittels Formalin inaktivierte Proben eingesetzt werden können. Wegen der nur teilweisen Übereinstimmung mit den zu behandelnden Chlamydien ist die Wirkung begrenzt.

Von der früher bzw. noch üblichen Therapie der mit Chlamydien infizierten Schafherden mit Tetracylin und seinen Derivaten ist bekannt, daß es nicht möglich ist, alle Tiere von Chlamydien zu befreien, so daß Jahr für Jahr besonders bei den Erstgebärenden nach wie vor Aborte in einer Rate von bzw. 20 - 40 % der Fälle auftreten.

Durch die erfindungsgemäße Immunisierung mit einer Autovakzine aus dem die Herde befallenden Erreger erreicht man eine Reduktion der Aborte auf unter 3 %.

Der Einsatz der Autovakzine in der Humangynäkologie verspricht eine ähnlich hohe Rate in der Prevention der Chlamydia trachomatis-verursachten Sterilitäten.

Autovakzine aus Chlamydien wurden im Versuchsstadium erfolgreich zur Bekämpfung der Pleuropneumonien bei Katzen auf Pleuraexudatbasis eingesetzt.

Das erfindungsgemäße Verfahren benutzt die in den Zellen- und Organschichten bzw. Exudaten befindlichen Chlamydien in den jeweiligen wirtsspezifischen bzw. eigenen Geweben und/oder Körperflüssigkeiten der befallenen Spezies (Katze, Schaf, Rind, Mensch).

Das jeweilige Material mit den intrazellulären Chlamydien wird entweder über abgestoßene Organteile (Placenten), Abschaben der Schleimhaut (Uterus) oder durch Punktion des Thorax (Pleuraexudate) isoliert.

Aus einer Plazenta von einem Lamm oder Kalb kann soviel mit Chlamydien befallenes Gewebe entnommen werden, daß es ausreichend ist, um eine Schaf- bzw. Rinderherde von 500 bzw. 100 Tieren mit jeweils 2,0 bzw. 5,0 ml 3 - 4mal zu impfen.

Die vorliegende Erfindung hat sich daher die Aufgabe gestellt, eine Autovakzine gegen Chlamydien zur Verfügung zu stellen, die spezifisch zu wirken und damit die Heilungschancen wesentlich zu verbessern in der Lage ist. Unter Autovakzine wird im Rahmen dieser Erfindung nicht nur eine Vakzine verstanden, die aus patienteneigenem Material gewonnen wurde, sondern auch solche Vakzine, die aus mit dem Patienten in längerem räumlichen Kontakt befindlichen anderen Patienten gewonnen wurde, bspw. in mehreren Tieren einer Herde oder Menschen einer Wohngemeinschaft oder gleicher Arbeitsstätte.

Die Lösung gelingt mit einem Verfahren gemäß Hauptanspruch. Vorteilhafte Ausgestaltungen dieses Verfahrens finden sich in den Unteransprüchen.

Das erfindungsgemäße Mittel stellt eine Autovakzine dar, die z. B. bei entsprechender Aufarbeitung subkutan und/oder peroral über die Schleimhaut verabreicht werden kann.

Das Verfahren geht von den in den Zellen oder Organschichten bzw. Exudaten der befallenen Organismen enthaltenen Chlamydien aus, unter Einschluß der sonstigen Bestandteile wie Zellmembranen und Proteine, Blutkörperchen sowie durch die Chlamydien hervorgerufene Toxoide etc. ohne Vermehrungsschritt der Chlamydien, im folgenden als Gewebematerial bezeichnet.

Durch die Behandlung des Gewebematerials bei Temperaturen über 50°C bis 90°C in Gegenwart quervernetzender Reagenzien werden alle enthaltenen Proteine individuell quervemetzt und dadurch denaturiert, d. h. es wird auf die Chlamydien und andere im Gewebematerial enthaltene Produkte in einer spezifischen Weise eingewirkt. Diese Behandlung führt überraschenderweise zur Bildung von stark antigen wirkenden Produkten, die bei ein oder gegebenenfalls mehrfacher Applikation an mit Chlamydien befallene Organismen zu einer protectiven Immunantwort führen, obwohl die Menge der Chlamydien in dem so behandelten und applizierten Gewebe-Material nur Bruchteile der im Organismus durch die Erkrankung vorhandenen Chlamydienmenge ausmacht. Das erfindungsgemäße Verfahren unterscheidet sich insoweit signifikant von der Herstellung von bakteriellen Autovakzinen, bei denen zunächst eine Bakterienprobe zur Beschaffung großer Mengen des antigenen Materials vermehrt und erst danach die Bakterien isoliert, abgetötet und in vergleichsweise großer Menge als Vakzine appliziert wird.

Durch die Quervemetzung und die Temperaturbehandlung kann sich das Gewebematerial verfestigen und es formen sich die eine anti-Erreger-spezifische Immunantwort induzierenden Partikel. Um eine zur Verarbeitung und zur Anwendung sinnvolle flüssige Konsistenz zu erhalten, wird dabei unter Rühren soviel pyrogenfreie physiologische Kochsalzlösung zugefügt, wie erforderlich ist.

Zur Herstellung einer subkutan zu verabreichenden Vakzine wird das so erhaltene Gemisch über Filter mit Porengrößen von unter 400 µm filtriert, um größere Komplexe abzutrennen. Das verflüssigte Mittel kann dem Patienten auch über die Schleimhäute des Mund/Rachenraumes (Gurgein) verabreicht werden.

Für die Quervernetzung der Proteine hat sich Formalin als besonders geeignet herausgestellt, dieses wird dem Gewebematerial in einer Menge von mindestens etwa 0,1 bis etwa 1,0 Volumenprozent in Form einer gesättigten Formalinlösung zugegeben. Paraformaldehyd, Glutaraldehyd, PLP (Perjodat-Lysin-Paraformaldehyd), Carbodiimide, insbesondere 1-Ethyl-3-(3-dimethylaminopropyl)-carbo-diimid oder 3',3'-Dimethylsuberimidat sind als weitere Beispiele für vernetzend wirkende Reagenzien zu nennen.

Die Denaturierungstemperatur in Anwesenheit quervernetzter Reagentien liegt bei über 50° C, vorzugsweise bei zwischen 80 und 85° C, wobei man diese Temperatur 1 - 5 Stunden, vorzugsweise etwa 2 Stunden aufrechterhält. Diese Zeit reicht aus, um die Chlamydien sowie chlamydienhaltige Zellen einer Denaturierung und Quervemetzung zu unterziehen, woraus eine Veränderung der Antigenität resultiert.

Diese Behandlung des Gewebematerials und der enthaltenen Antigene (Erreger) führen zu einer Veränderung der antigenen Eigenschaften durch Denaturierung der Proteine (Wärme) bei gleichzeitiger Quervemetzung (Formalin bzw. Formaldehyd oder Para-Formaldehyd oder Phenol bzw. seine Derivate). Diese Denaturierung plus Quervemetzung führt dazu, daß das Antigen (der Erreger) dem Immunsystem in einer anderen als der nativen Weise zugänglich wird. Daraus ergibt sich, daß auch Erreger, die im nativen Zustand möglicherweise unerkannt bleiben oder eine nicht adäquate Form der Immunantwort induzieren (chronisch inflammatorischer Verlauf o. ä.), abgewehrt werden können. Es wird angenommen, daß neben den Erregern selbst auch andere körpereigene oder durch die Infektion gebildete Bestandteile/Rezeptoren der Zelle denaturiert, quervernetzt und für den Kontakt mit den Antikörper bildenden Zellen des Immunsystems aufbereitet und zur Verfügung gestellt werden und daß dadurch die erhöhte Aktivität der erfindungsgemäßen Autovakzine bewirkt wird.

Diese Überlegungen wurden durch umfangreiche Untersuchungen sowohl mit tierischen als auch zum Teil mit menschlichen Patienten gestützt. Größtenteils handelte es sich dabei um chronisch persistierende oder rezidivierende Infektionen. Man kann also davon ausgehen, daß das Antigen dem Immunsystem grundsätzlich zugänglich war. Nach Applikation des Antigens in denaturierter und quervernetzter Form kam es in den meisten Fällen in weniger als vier Wochen zur Heilung der Infektion. Dies kann nur durch die beschriebene veränderte Präsentationsform des Antigens durch Wärme und Formaldehyd erklärt werden.

### Beispiel I

Aus der frischen Plazenta eines mit Chlamydien infizierten abortierten Lamms bzw. Kalbs werden 300 bzw. 500 g Gewebematerial entnommen und zunächst ca. 60 min. bei 95° C erhitzt. Anschließend werden die Placentateile mit einem Zerkleinerungsgerät soweit wie möglich zerkleinert und danach durch ein steriles Sieb (Porenweite 400 µm) oder über einen sterilen Trichter in eine sterile Flasche durchpassiert.

Diese Masse wird mit steriler pyrogenfreier physiologischer NaCl-Lösung ca. 1 : 3 verdünnt, mit 0,3 Vol.-% Formalin versetzt und erneut 2 Std. bei 80° C erhitzt, 24 Std. bei 37° C inkubiert und anschließend auf Sterilität kontrolliert. Bei negativer Sterilitätskontrolle ist die Autovakzine einsatzbereit.

Die erhaltene Vakzine ist ausreichend, um eine mit Chlamydien infizierte Schafoder Rinderherde von 500 bzw. 100 Tieren mit jeweils ca. 2,0 bzw. 5,0 ml 3 - 4mal zu impfen. So behandelte Muttertiere zeigen eine Abortrate von unter 3 % verglichen mit unbehandelten Tieren, die eine Abortrate von 20 - 40 % aufweisen.

### Beispiel II

Zur Behandlung von Chlamydia (psittaci, pneumoniae oder trachomatis) Infektionen bei Menschen wird entweder ein Pleuraexudat oder bei Frauen ein Schleimhautabstrich aus dem Uterus entnommen. In letzterem Fall werden Proben mehrerer Abstriche gesammelt und bei - 20° C zwischengelagert, bis die notwendige Gesamtmenge von 5 - 6 ml erreicht ist, um zur Immunisierung des Patienten auszureichen. Diese Probe wird zerkleinert und analog zu Beispiel I behandelt.

Bei Verfügbarkeit entsprechender Materialmengen können Chlamydia-pneumoniae-Autovakzinen zur Beeinflussung entzündlicher, durch pneumoniae bedingte Prozesse der Coronarflüssigkeit verwendet werden.

Die Applikation beim Menschen erfolgt subkutan unter die Abdominalhaut in 4 Impfungen ä 1,5 bis 3 ml. Ist genügend Impfstoffmasse (Pleuraexudat) vorhanden, empfiehlt es sich, parallel dazu eine perorale Applikation von 5 - 10 ml/Tag über die ersten 10 Tage durchzuführen.

Schocksituationen sind nicht zu erwarten, da das die Chlamydien umgebende Protein speziesspezifisch ist. Beim Menschen empfiehlt sich trotzdem vor der ersten Sc-Applikation ein Allergietest.

## Patentansprüche

1. Verfahren zur Herstellung von Autovakzinen aus patienteneigenem Material oder aus mit dem Patienten in längerem räumlichen Kontakt befindlichen anderen Patienten gewonnenem Material zur Behandlung von Chlamydiosen von Säugetieren und Menschen, **dadurch gekennzeichnet, dass** man Chlamydien enthaltendes Gewebematerial, ohne Vermehrungsschritt für die Chlamydien, in Gegenwart von Proteine quervemetzenden Mitteln auf Temperaturen von über 50 °C bis 90 °C 1 - 5 Stunden erwärmt und in eine applizierbare flüssige Konsistenz bringt.

2. Verfahren nach Anspruch 1, **dadurch gekennzeichnet, dass** man als proteinvemetzende Mittel Formalin, Paraformaldehyd, Glutaraldehyd, PLP (Perjodat-Lysin-Paraformaldehyd), Carbodiimide, insbesondere 1-Ethyl-3-(3-dimethylaminopropyl)-carbo-diimid oder 3',3'-Dimethylsuberimidat zusetzt und die Temperatur 2 Stunden bei 80 - 85 °C hält.

3. Verfahren nach Anspruch 1 oder 2, **dadurch gekennzeichnet, dass** man pyrogenfreie physiologische Kochsalzlösung zur Verflüssigung zufügt.

4. Verfahren nach einem der Ansprüche 1 bis 3, **dadurch gekennzeichnet, dass** man die Vakzine durch ein Filter mit einer Porenweite von unter 400 µm passiert.

5. Verfahren nach einem der Ansprüche 1 bis 4, **dadurch gekennzeichnet, dass** dem Gewebematerial 0,1 bis 1,0, vorzugsweise 0,5 Volumenprozent, einer gesättigten Formalinlösung zugegeben werden.

6. Autovakzine zur Behandlung von Chlamydiosen von Säugetieren und Menschen, enthaltend gemäß Anspruch 1 bis 5 quervernetztes Gewebematerial.

## Claims

1. Process for the preparation of autovaccines which are obtained from the patient's own materials or from material obtained from other patients present in comparatively long spatial contact with the patient for the treatment of chlamydioses of mammals and humans, **characterised in that** one heats chlamydia-containing tissue material, without propagation step for the chlamydia, in the presence of protein cross-linking agents for 1 to 5 hours to temperatures of above 50 °C to 90 °C and brings to an administerable liquid consistency.

2. Process according to claim 1, **characterised in that**, as protein cross-linking agent, one adds formalin, paraformaldehyde, glutaraldehyde, PLP (periodate-lysine-paraformaldehyde), carbodiimides, especially 1-ethyl-3-(3-dimethylaminopropyl)-carbodiimide or 3,3'-dimethylsuberimiidate and maintains the temperature for 2 hours at 80 - 85 °C.

3. Process according to claim 1 or 2, **characterised in that** one adds pyrogen-free physiological common salt solution for the liquefaction.

4. Process according to one of claims 1 to 3, **characterised in that** one passes the vaccine through a filter with a pore width of below 400 µm.

5. Process according to one of claims 1 to 4, **characterised in that** to the tissue material one adds 0.1 to 1.0, preferably 0.5 volume percent of a saturated formalin solution.

6. Autovaccine for the treatment of chlamydioses of mammals and humans containing tissue material cross-linked according to claim 1 to 3.

## Revendications

1. Procédé de préparation d'autovaccins à partir de matière propre à un patient ou à partir de matière extraite d'autres patients se trouvant en contact spatial prolongé avec le patient, pour traiter les chlamydioses chez les mammifères et l'humain, **caractérisé en ce que**, on chauffe la matière tissulaire contenant les chlamydies sans étape de multiplication pour les chlamydies, en présence d'agents réticulant les protéines, à des températures supérieures à 50°C allant jusqu'à 90°C pendant 1 à 5 heures et que l'on amène à une consistance liquide pouvant être appliquée.

2. Procédé selon la revendication 1, **caractérisé en ce que** l'on ajoute en tant qu'agents réticulant les protéines, le formol, le paraformaldéhyde, le glutaraldéhyde, le PLP (periodate-lysine-paraformaldéhyde), les carbodiimides, en particulier le 1-éthyl-3-(3-diméthylaminopropyl)-carbodiimide ou le 3',3'-diméthylsubérimidate, et on les maintient à la température de 80 à 85°C pendant 2 heures.

3. Procédé selon la revendication 1 ou 2, **caractérisé en ce que** l'on ajoute une solution saline physiologique apyrogène pour fluidification.

4. Procédé selon l'une quelconque des revendications 1 à 3, **caractérisé en ce que** l'on fait passer les vaccins à travers un filtre ayant une taille de pores inférieure à 400 µm.

5. Procédé selon l'une quelconque des revendications 1 à 4, **caractérisé en ce que** 0,1 à 1,0, de préférence 0,5 pourcent en volume d'une solution de formol saturé sont ajoutés à la matière tissulaire.

6. Autovaccins pour traiter les chlamydioses chez les mammifères et chez l'humain, contenant une matière tissulaire réticulée selon la revendication 1 à 5.
